# EUROPEAN PATENT SPECIFICATION

(11) **EP 2 468 197 B1**
(45) Date of publication and mention of the grant of the patent: **21.11.2012**
(21) Application number: 10196866.7
(22) Date of filing: 23.12.2010
(51) Int. Cl.: A61B 17/70

(54) **Stabilization device for stabilizing vertebrae or bone parts**
Stabilisierungsvorrichtung zur Stabilisierung von Wirbeln oder Knochenteilen
Dispositif de stabilisation permettant de stabiliser les éléments osseux ou vertèbres

(43) Date of publication of application: 27.06.2012
(73) Proprietor: Biedermann Technologies GmbH & Co. KG, 78166 Donaueschingen (DE)
(72) Inventor: Biedermann, Lutz, 78048, VS-Villingen (DE); Matthis, Wilfried, 79367, Weisweil (DE); Dannecker, Berthold, 78112, St. Georgen (DE)
(74) Representative: Prüfer & Partner GbR European Patent Attorneys

(56) References cited:
- EP-A1- 2 070 485
- EP-A1- 2 201 903
- DE-A1- 19 912 364
- DE-C1- 4 110 002
- US-A- 5 873 878

## Description

The invention relates to a stabilization device for stabilizing vertebrae or bones that includes a bone anchoring device and at least two stabilization rods of different diameter.

For stabilizing the spinal column bone anchoring devices are known that comprise a shaft to be anchored in the bone and a head to be connected to a rod. Usually, a rod connects several bone anchoring devices. Depending on the medical indication and the region of the spine that is to be stabilized rods with different diameters are required. The diameter of the rods ranges from 3mm to more than 6mm. Generally, the diameter of the rod used in the lower part of the spine is larger than the diameter of the rod used in the upper part of the spine. For example, in the cervical-thoracic region of the spine rods with a diameter of 3mm to 3.5mm, in the transitional zones between the cervical-thoracic and the thoracic-lumbar region rods with a diameter of 3.5mm to 4.5mm, in the thoracic-lumbar region rods of usually 4.5mm to 5.5mm and the lumbar-sacral region rods with a diameter of 5mm to 6.35mm are necessary.

For each rod of a certain diameter specific bone anchoring devices, such as pedicle screws, are required. They differ from each other in particular by the size of the recess into which the rod is inserted. The provision of different bone anchoring devices increases the costs and renders spinal surgery more complicated for surgeon.

US 5,873,878 discloses an anchoring member for attachment to a vertebra and for use with a first rod having a first diameter and a second rod having a second, smaller diameter. The anchoring member comprises an insert member which can be inserted into the head of the anchoring member so as to allow the insertion of a rod with a smaller diameter.

EP 2 070 485 Al describes a monoaxial bone anchoring device and a polyaxial bone anchoring device each of which can be used with rods of different diameters.

EP 2 201 903 A1 discloses a receiving part for receiving a rod for coupling the rod to a bone anchoring element. The receiving part includes a receiving part body with a rod receiving portion with a channel for receiving the rod, and a head receiving portion for accommodating a head of the bone anchoring element, the head receiving portion having an open end and being flexible so as to allow introduction and clamping of the head, the head receiving portion having an exterior surface with a curved portion; and a locking ring embracing the head receiving portion, and wherein the locking ring has an interior surface with a curved portion, which presses against the curved portion of the exterior surface of the head receiving portion to clamp the head.

DE 199 12 364 A1 discloses a pedicle screw for fixing a stabilization rod along the vertebral column, comprising a thread section that can be screwed into the bone material of a vertebra, and a head having a recess of substantial U-shape and limited by U-legs for inserting the stabilization rod, and an inner thread at the U-legs for fixing the stabilization rod in the recess by virtue of a fixing screw, characterized in that the inner thread is an asymmetrical saw tooth thread such that those thread flanks, which bear a load upon pulling tight the fixing screw reveal a negative angle.

DE 41 10 002 C1 discloses a vertebral column correcting and stabilising implant which has a screw head retainer wall with two parallel, protruding edges pointing against the threaded rod.

It is the object of the invention to provide a stabilization device for vertebrae or bones that is further improved with respect to the modularity of the system.

The object is solved by a stabilization device according to claim 1. Further developments are given in the dependent claims.

With the stabilization device a modular system can be provided that allows to combine various anchoring elements with any suitable receiving part and any suitable rod on demand depending on the actual clinical requirements. This reduces the costs of polyaxial screws, reduces the inventory and gives the surgeon a substantial choice of implants.

The assembly of the stabilization device can be carried out by any specialist, for example, by the surgeon or any personnel assisting him before or during surgery.

The anchoring device has an advantage in that it provides a safe clamping of any of the rods having a different diameter. The clamping force does not depend on the diameter of the rod. Also, the clamping of rods of different diameter is achieved in a stepless manner.

Furthermore, the bone anchoring device is constructed to minimize the number of parts. Hence it does not require additional parts to allow the fixation of different rods.

The stabilization device can be used for example for the correction of scoliosis in children. When a child having a scoliosis correction grows up, it may be necessary to adapt the scoliosis correction device. For example, it may be necessary to use other rods with a greater diameter as those originally inserted. With the stabilization device according to the invention it is possible to replace the originally used rods in a second surgery with the bone anchors remaining anchored in the vertebrae.

Further features and advantages of the invention will become apparent from the description of embodiments by means of the accompanying drawings.

In the drawings:
- Fig. 1: shows a perspective exploded view of the stabilization device with a first rod according to a first embodiment.
- Fig. 2: shows a perspective view of the stabilization device of Fig. 1 in an assembled state.
- Fig. 3: shows a cross-sectional view of the stabilization device of Fig. 2 with the first rod in the assembled state, the section being taken perpendicular to the rod axis.
- Fig. 4: shows a side view of the stabilization device of Fig. 3.
- Fig. 5: shows a perspective view of a locking ring of the receiving part of the stabilization device according to Figs. 1 to 4.
- Fig. 6: shows a side view of the locking ring of Fig. 5.
- Fig. 7: shows a top view of the locking ring of Fig. 5.
- Fig. 8: shows a shows a cross-sectional view of the stabilization device of Fig. 2 with a second rod in the assembled state, the section being taken perpendicular to the rod axis.
- Fig. 9: shows a side view of the stabilization device of Fig. 8.
- Fig. 10: shows a perspective view of a locking ring of the receiving part of a the stabilization device according to a second embodiment.
- Fig. 11: shows a side view of the locking ring of Fig. 10.
- Fig. 12: shows a top view of the locking ring of Fig. 10.
- Fig. 13: shows an cross-sectional view of an enlarged portion of the rod support of the locking ring according a modified second embodiment.
- Fig. 14a) to c): show cross-sectional views of an enlarged portion of the rod support of the locking ring according a further modified second embodiment with rods having different diameters.
- Fig. 15a) to c): show cross-sectional views of an enlarged portion of the rod support of the locking ring according a still further modified second embodiment with rods having different diameters.

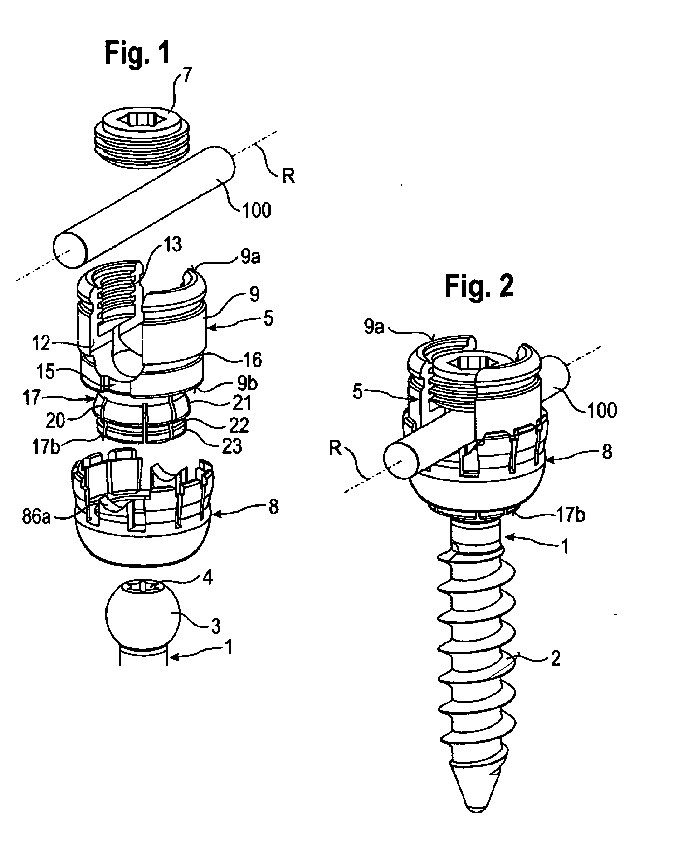

As shown in Figs. 1 and 2, the stabilization device according to a first embodiment includes a bone anchoring element 1 in the form of a bone screw having a threaded shaft 2 and a head 3 with a curved surface portion. In this embodiment the head is spherical segment-shaped. The head 3 has a recess 4 for engagement with a tool. The stabilization device also comprises a receiving part 5 for receiving a first rod 100 to connect it to the bone anchoring element 1. Further, a fixation element 7 in the form of an inner screw is provided for fixing the rod 100 in the receiving part 5. The stabilization device includes a locking ring 8 for locking the head 3 in the receiving part 5. At least one further rod (not shown) is provided that has a diameter different from the diameter of the first rod. The rods have a circular cross-section. Also, the rods have a substantially smooth surface.

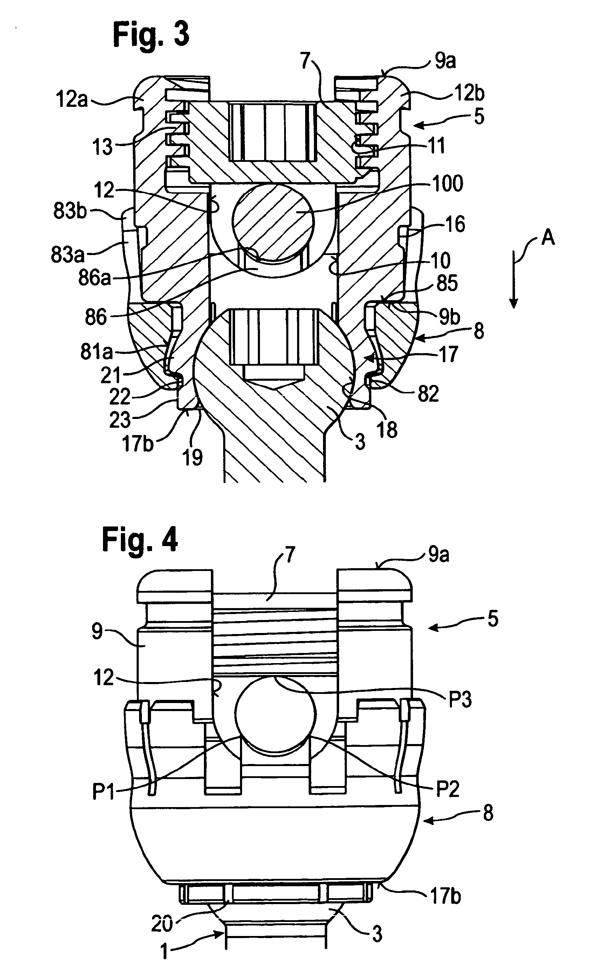

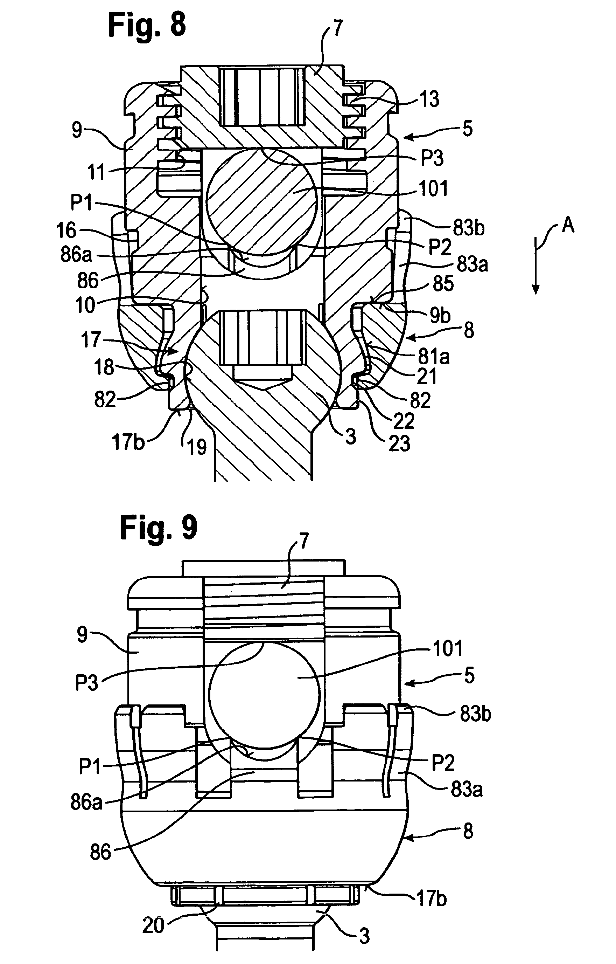

The receiving part 5 will be explained with reference to Figs. 1 to 4. It comprises a rod receiving portion 9, which is substantially cylindrical and which has a first end 9a and an opposite second end 9b. A coaxial first bore 10 is provided at the second end 9b as shown in Figs. 3 and 8. The diameter of the first bore 10 is smaller than the diameter of the head 3 of the bone anchoring element. The rod receiving portion 9 further has a coaxial second bore 11 extending from the first end 9a to a distance from the second end 9b. The diameter of the second bore 11 is larger than that of the first bore 10. A substantially U-shaped recess 12 extends from the first end 9a in the direction of the second end 9b in the rod receiving portion, the diameter of the recess 12 being larger as the diameter of any of the rods so that the rod 100 and any other rod of the stabilization device can be placed in the recess and can be guided therein. By means of the recess 12 two free legs are formed on which an internal thread 13 is provided. The internal thread can be a metric thread, a flat thread, a negative angle thread, a saw-tooth thread, or any other thread form. Preferably, a thread form such as a flat thread or negative angle thread is used, which prevents splaying of the legs 12a, 12b when the inner screw 7 is screwed-in. The depth of the recess 12 is such that the rod 100 oreach of the further rods of the stabilization device and the inner screw 7 can be inserted between the legs.

As can be seen in Fig. 1, cut-outs 15 are provided in the rod receiving portion on either end of the channel formed by the recess 12.

In the outer surface of the rod receiving portion 9 a groove 16 is provided, which extends in a circumferential direction and serves for engagement with a portion of the locking ring 8.

At the side of the second end 9b the receiving part 5 comprises a head receiving portion 17 providing an accommodation space for the head 3 of the bone anchoring element 1. The head receiving portion 17 has a greatest outer diameter, which is smaller than the greatest outer diameter of the rod receiving portion 9. An internal hollow section 18 forms a seat for the head 3 of the bone anchoring element 1 and is open via the opening 19 to the free end 17b of the head receiving portion. The hollow section 18 is adapted in its shape to the shape of the head 3, in the embodiment shown, it is a spherical section to accommodate the spherical head 3. Furthermore, the hollow section 18 is configured to encompass the head 3 of the bone anchoring element from the side covering a region including the largest diameter of the head 3.

A plurality of slits 20 are provided in the head receiving portion 17, which are open to the free end 17b. The slits 20 render the head receiving portion 17 flexible so that it can be compressed to clamp and finally lock the head 3 in the hollow internal portion 18 by means of friction. The number and size of slits 20 is provided depending on the desired flexibility of the head receiving portion 17. The flexibility of the head receiving portion 17 is such that the head 3 of the anchoring element can be inserted by expanding the head receiving portion and that it can be clamped by compressing the head receiving portion.

The outer surface of the head receiving portion 17 has a first section 21, with an outer diameter which increases towards free end 17b, for example in an outwardly curved manner. Adjacent to the first section 21, there is a circumferential groove 22, which is recessed with respect to the first section 21 and which serves for engagement with portion of the locking ring 8. Adjacent the groove 22, there is a third portion 23 of the head receiving portion with a substantially cylindrical outer surface. The third portion 23 is configured to cooperate with a portion of the locking ring to enhance the clamping effect of the locking ring.

The locking ring 8 will now be described with reference to Figs. 1 to 7. The locking ring 8 is substantially cylindrical and has an upper end 8a and a lower end 8b. In the mounted state the upper end 8a is oriented in the direction of the first end 9a of the rod receiving portion and the lower end 8b is oriented towards the free end 17b of the head receiving portion. As can be seen in Figs. 3 and 8, an inner surface portion 8 1 a is provided near the upper end, which cooperates with the first outer surface portion 21 of the head receiving portion to exert a compression force onto the head receiving portion 17.

At the lower end 8b, the locking ring comprises an inwardly projecting edge 82, the inner diameter of which is smaller than the inner diameter of the other portions of the locking ring. The inwardly projecting edge 82 is configured to engage the groove 22 of the head receiving portion.

The locking ring 8 further has upwardly extending wall portions 83a, which are separated from each other by slits 84. The upwardly extending wall portions 83a are arranged at the outer circumference of an inner circumferential shoulder 85 of the locking ring and render the upper portion of the locking ring flexible. The number and size of the slits and the thickness of the wall portions 83a are configured such that a desired flexibility is obtained. At the free end the wall portions 83a comprise engagement sections 83b which are shaped so as to engage the groove 16 provided on the outer surface of the rod receiving portion 9.

The locking ring 8 is sized in such a way with respect to the head receiving portion 17 that the head receiving portion can expand within the locking ring to allow the introduction of the head 3 when the locking ring is a the first position as shown in Fig. 3.

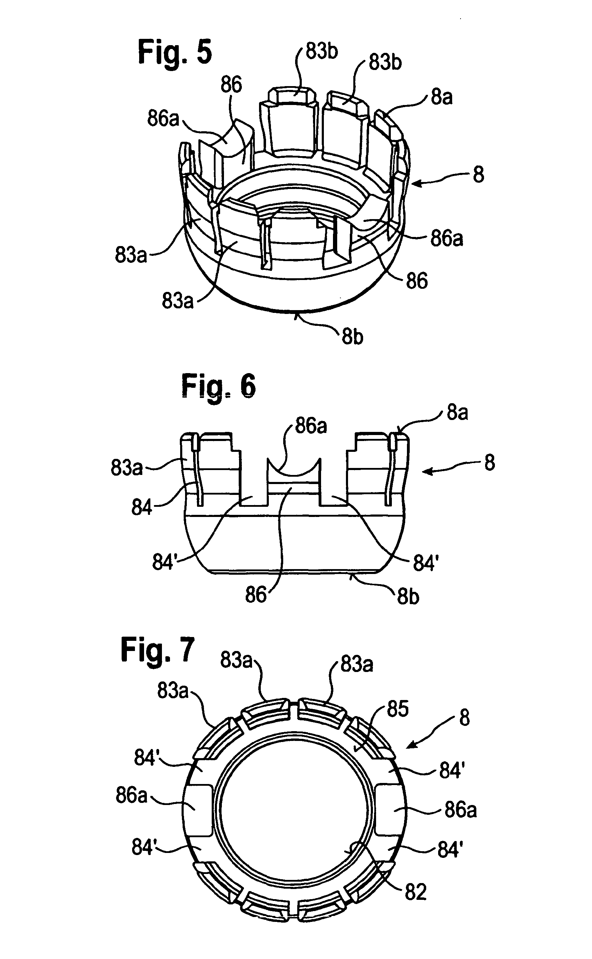

Two projections 86, which are located diametrically opposite to each other, are formed in the upper portion of the locking ring. The projections 86 have such a height that they project above the bottom of the substantially U-shaped recess 12 and extend into the cut-outs 15, when the locking ring 8 is in a position, in which the head 3 is not yet locked as shown in Figs. 5. The projections are separated from the upwardly extending wall portions 83a by slits 84' the size of which is designed such that a distance of the wall portions 83a to the left and right of the projections 86 is greater than the width of the cut-outs 15 in the receiving part. The locking ring is arranged in such a manner around the head receiving portion 17 of the receiving part 5, that the projections 86 are located at the positions of the recess 12. By means of this, the projections 86 prevent the locking ring from rotating when the rod is not inserted.

As can be seen in particular in Figs. 3 to 6 a free end surface 86a of the projections is concave. In the first embodiment the free end surface 86a is formed as a cylindrical recess with a cylinder radius that is smaller than the radius of the first rod 100 and smaller than the radius of any of the further rods. The width of the projections 86 in the region of the free end surface 86a is smaller than the diameter of any of the rods that an be inserted. Hence, when the first rod 100 is inserted into the U-shaped recess 12 and placed onto the projections 86, the first rod 100 contacts the free end surface 86a of the projections 86 at two contact areas P1, P2 that are substantially formed as line contacts extending in the direction of the rod axis R.

The contact areas are located slightly apart from the free edges of the end surfaces 86a towards the bottom of the free end surface.

When the inner screw 7 is inserted and tightened, it contacts the rod 100 along a third contact area P3 that is on the top of the rod 100. The third contact area P3 is also substantially a line contact, which extends in the direction of the rod axis R.

The flexibility of the head receiving portion 17 and the size of the head receiving portion at the open end 17b allows to mount the locking ring 8 by assembling it from the free end 17b onto the head receiving portion 17.

The inner screw 7 has a thread corresponding to the internal thread 13. If a thread form, which prevents the legs from splaying is used, a single fixation element such as the inner screw 7 is sufficient. This reduces the size of the bone anchoring device in a radial direction. Other fixation elements such as, for example, an outer nut are also possible.

The stabilization device comprises a second rod 101 which has a diameter that is greater that the diameter of the first rod 100 as shown in Figs. 8 and 9. When the second rod 101 is inserted into the receiving part, it rests on the free edges of the end surface 86a of the projections of the locking ring. Hence, the contact areas P1 and P2 are slightly farther apart from each other compared to the contact areas of the first rod 100.

The dimensions of the projections and the concave free end surface 86a and the diameter of the rod as well as the length and shape of the inner screw 7 are such that the rods are clamped along three contact lines P1, P2 and P3. The rod is in a stable position when it is clamped in this way, similar to a three-point fixation. By means of this a secure multi-line contact along the rod is achieved at each projection 86. It should be understood that the contact lines are not infinitesimally thin lines, but are lines which have a certain thickness according to the contact that is macroscopically generated. Hence, a safe fixation independently of the diameter of the rod is provided.

The diameter of the rods that can be used with the anchoring device may vary between a largest diameter and a smallest diameter that are defined geometrically in such a way that the rod has in any case two lines of contact with the free end surface of the projections.

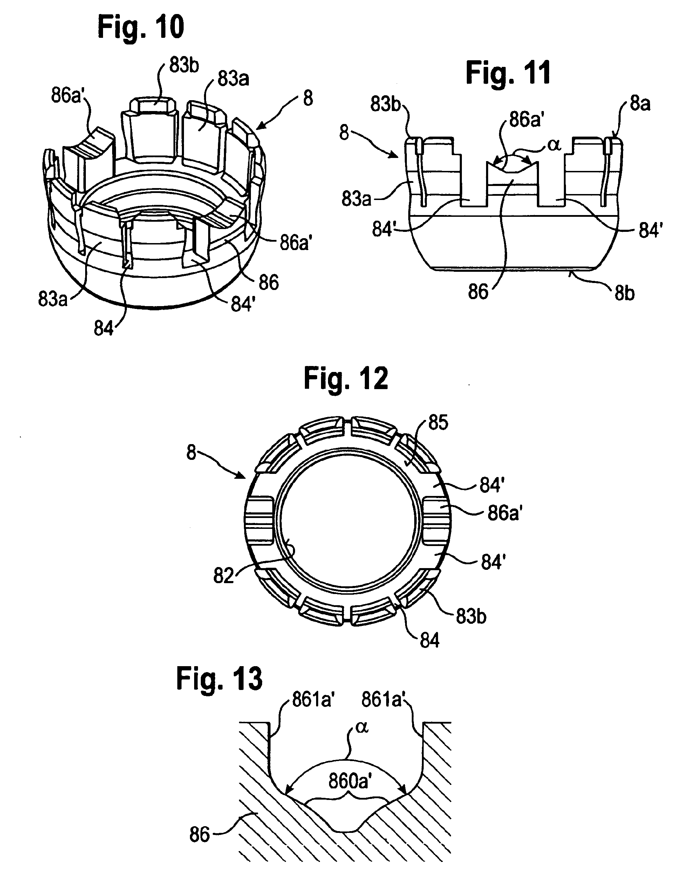

A second embodiment of the stabilization device differs from the first embodiment by the shape of the projections provided on the locking ring. All other portions of the stabilization device are the same and have the same reference numerals. Their description is not repeated. As shown in Figs. 10 to 12, the free end surface 86a' of the projections according to the second embodiment form grooves having a substantially V-shaped cross-section. The bottom of the V can be straight or rounded. An angle α included by the V-shape is such that a rod with a diameter that is smaller than the largest diameter of the V-shaped groove contacts the groove at two opposite contact areas P1, P2 extending in a direction parallel to the rod axis R. As in the first embodiment, the contact areas are substantially line contacts.

As shown in Fig. 13, in a modified second embodiment, the bottom of the V-shaped groove has inwardly curved portions 860a' and straight sidewalls 861a'.

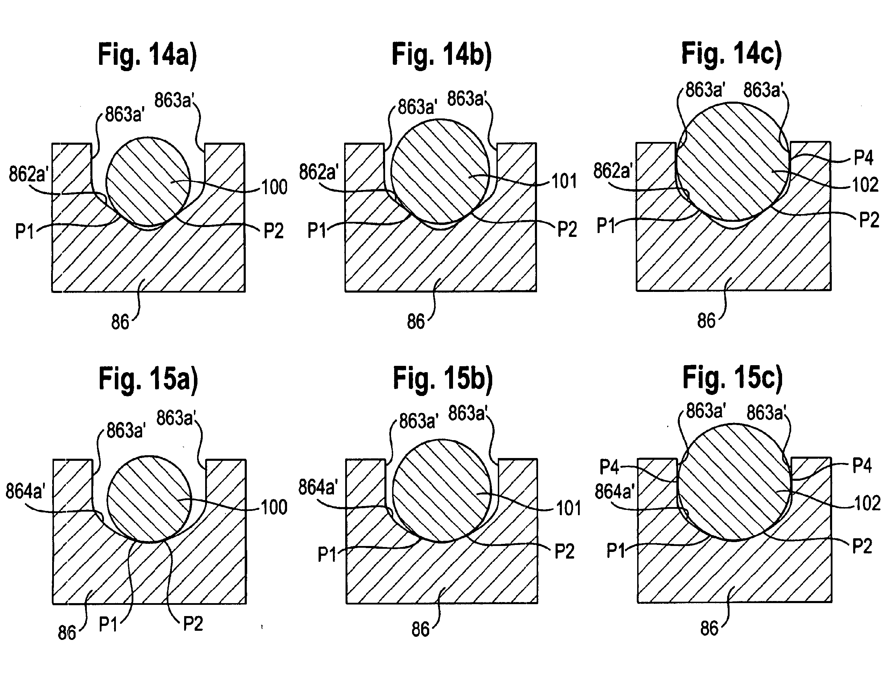

In a further modification, as shown in Figs. 14a) to c) the V-shaped groove has additionally straight side walls 863a'.

In a still further modification as shown in Figs. 15a) to 15c) the V-shaped groove has concavely curved sidewalls 864a' of the V and straight sidewalls 863a'. Also in the modified embodiments the rod rests on the free end surface 86a' along two contact areas P1, P2.

Figs. 14a) to 14c) and Figs. 15a) to 15c) show schematically the contact of rods 100, 101, 102 with increasing diameter. The rods in each case rest in the V-shaped groove on two points or, when seen three-dimensionally, on two contact areas P1,P2 in the form of line contacts. From above the rods are clamped by the inner screw of the fixation element. Hence, each rod is in a stable clamping position similar to a three-point clamping. If a large rod is used, the rod may touch the V-shaped groove also in the region of the straight side walls at a contact area P4.

The receiving part 5, the locking ring 8, the inner screw 7 and the bone anchoring element 1 are made of a bio-compatible material, for example of titanium or stainless steel or a bio-compatible alloy such as Nitinol or a bio-compatible plastic material, such as PEEK. Parts can all be made of the same or of different materials.

The function of the locking ring that is the same for all embodiments will now be explained with referenced Figs. 3 and 8. As shown in Fig. 3 and 8, a first position of the locking 8, which is the insertion position and in which the locking ring 8 is latched with respect to the receiving part 5, is defined in such a way that the inwardly projecting edge 82 engages groove 22 at the outer surface of the head receiving portion 17. The inner diameter of the inwardly projecting edge 82 is larger than the outer diameter of the head receiving portion 17 at the position of the groove 22, so as to allow an expansion of the head receiving portion 17, when the head 3 is introduced. In the first position, the locking ring 8 is additionally held by a clamping force between the rod receiving portion 9 of the receiving part 5 and the flexible wall portions 83a of the locking ring which are slightly bent outwards.

When the locking ring is in the first position, the head receiving portion 17 is not compressed. In this position, the introduction of the screw head is possible. In the first position, the locking ring is prevented from moving upwards towards the first end 9a of the rod receiving portion, since it abuts with the shoulder 85 against the second end 9b of the head receiving portion and with the inwardly projecting edge 82 against the upper wall 16b of groove 16. The abutment of the locking ring against the second end 9b and against the upper wall of groove 16 holds the locking ring 8 in place against upward movement. Since the inner diameter of the locking ring is larger than the outer diameter of the head receiving portion in a non-compressed state, an expansion of the head receiving portion 17 into the space within the locking ring is possible. In the first position, the head can freely pivot.

A second position (not shown), in which the locking ring is latched with respect to the receiving part 5 and which is the pre-locking position is achieved by shifting the locking ring 8 as indicated by the arrow A towards the free end 17b of the head receiving portion until the engagement portions 83b of the flexible wall portions 83a resiliently snap into the groove 16 provided at the rod receiving portion 9.

In the second position the inner inclined surface 81a of the locking ring presses against the first outer surface portion 21 of the head receiving portion so as to compress the head receiving portion 17 to clamp the head 3 within the hollow internal portion 18 without fully locking the head. In addition, the inwardly projecting edge 82 presses against the third portion 23 of the head receiving portion 17 resulting in an additional clamping force. Thereby, clamping of the head can be effected not only from above or the side of the head 3 but also from a region of the lower portion of head 3. Under conditions arising during surgery, the angular position of the bone anchoring element 1 with respect to the receiving part 5 is maintained and can be loosened only by exerting an additional force onto the receiving part of the screw element. In this pre-locked position the bone anchoring element cannot be removed from the receiving part but angulation of the screw element is possible.

A third position (not shown), which is the locking position, is achieved by shifting the locking ring further downwards until the screw head 3 is finally locked within the head receiving portion 17. The inner surface 81a of the locking ring engages the outer surface of the first portion 21 of the head receiving portion 17 in such a way that the head 3 is locked by compression of the head receiving portion. In addition, the inwardly projecting edge 82 further compresses the head receiving portion at the lower portion 23, thereby enhancing the locking force.

The bone anchoring device is preassembled as follows. First, the locking ring 8 is mounted onto the receiving part 5 from the free end 17b. This can be done, for example, by the manufacturer. Preferably, the locking ring 8 is in the first position shown in Figs. 3 an 8 in which it is latched by engagement of the inwardly projecting edge 82 with the groove 22. Thereafter, the head 3 of the anchoring element 1 is introduced from the free end 17b into the hollow internal portion 18 of the head receiving portion 17. Thereafter, the locking ring is moved relative to the receiving part downwards, so that the inwardly projecting ring 82 slides out of the groove 22 and the engagement portions 83b of the flexible wall portions 83a snap into groove 16, whereby the second position.

In use during surgery, the preassembled bone anchoring device comprising the receiving part, the bone anchoring element and the locking ring in the pre-locking position, is screwed into the bone. The recess 4 of the head can be accessed with a screw tool through the first bore 10.

A rod is selected that has a diameter suitable for the specific clinical application and inserted into the recess 12 until it is supported by the free end surface of the projections 86. Hence, with the stabilization device a modular system is provided that allows to select and assemble a suitable anchoring element and a suitable rod.

To correctly align the receiving part with respect to the rod, the receiving part is pivoted. Once the correct position of the rod with respect to other bone anchoring devices is achieved, the inner screw 7 is tightened. Since the rod 6 abuts onto the projections 86 of the locking ring, the locking ring 8 is shifted downward into the third position which is the locking position. When the locking ring 8 is moved towards the free end 17b of the head receiving portion, it compresses the head receiving portion, thereby locking the head. Final tightening of the inner screw locks the rod and the head simultaneously.

Further modifications of the embodiment shown are possible. For example, the locking ring can have another shape. The pre-locking function can be realized otherwise.

For the anchoring element, all kinds of known anchoring elements such as screws, nails, hooks, cannulated screws, anchoring elements with separated head and shaft that can be connected to each other can be used.

The rods may also have a curvature along the rod axis.

The head receiving portion can have an inclined open end or can be otherwise asymmetric to allow a greater angulation of the head in one direction.

The outer surface of the head receiving portion and the inner surface of the locking ring can have other shapes which allow a compression of the head receiving portion when the locking ring is shifted downwards.

## Claims

1. Stabilization device for stabilizing vertebrae or other bones including at least two rods (100, 101) having a different diameter;
an anchoring element (1) having a shaft (2) for anchoring in the bone and a head (3);
a receiving part (5) for connecting one rod from the at least two rods to the anchoring element;
the receiving part comprising
a top end (9a) and a bottom end (17b);
a rod receiving portion (9) with a channel (12) for receiving one of the rods (100,101), and
a head receiving portion (17) for accommodating a head (3) of the bone anchoring element, the head receiving portion having an open end (19, 17b) and being flexible so as to allow introduction and clamping of the head; and
a locking ring (8) embracing the head receiving portion (17), wherein the head is clamped by pressing onto the locking ring with the rod to move the locking ring towards the open end (17b);
wherein the locking ring (8) has a surface portion (86a, 86a') configured to contact one
of the rods (100, 101) in a circumferential direction at at least two distinct contact areas (P1, P2).

2. The stabilization device of claim 1, wherein the surface portion is shaped such that the contact areas (P1, P2) extend along the rod axis (R).

3. The stabilization device of claim 1 or 2, wherein the surface portion (86a) is curved with a radius of curvature that is smaller than a radius of curvature of each of the rods (100, 101).

4. The stabilization device of one of claims 1 to 3, wherein the surface portion (86a) is formed through a cylindrical recess with a diameter smaller than the diameter of any of the rods (100, 101).

5. The stabilization device of one of claims 1 to 3, wherein the surface portion (86a') is substantially V-shaped.

6. The stabilization device of claim 5, wherein the side walls (860a',864a')of the base which form the V-shape are curved.

7. The stabilization device of claim 5 or 6, wherein the surface portion further includes straight sidewalls (863a').

8. The stabilization device of one of claims 1 to 7, wherein the surface portion is shaped such as to clamp a plurality of rods with different diameters in a stepless manner.

9. The stabilization device of one of claims 1 to 8, wherein the rod has a substantially circular cross section.

10. The stabilization device of one of claims 1 to 9, wherein the locking ring has two projections (86) that are offset by 180° from each other and wherein each of the projections comprises the surface portion configured to contact each of the rods in a circumferential direction at at least two distinct contact areas.

11. The stabilization device of claim 10, wherein the projections (86) extend into the channel (12).

## Patentansprüche

1. Stabilisierungsvorrichtung zum Stabilisieren von Wirbeln oder anderen Knochen, die umfasst:
mindestens zwei Stäbe (100, 101), die einen unterschiedlichen Durchmesser haben;
ein Verankerungselement (1) mit einem Schaft (2) zum Verankern in den Knochen und einen Kopf (3);
ein Aufnahmeteil (5) zum Verbinden eines Stabs von den mindestens zwei Stäben mit dem Verankerungselement;
wobei das Aufnahmeteil aufweist:
ein oberes Ende (9a) und ein unteres Ende (17b);
einen Stabaufnahmeteil (9) mit einem Kanal (12) zum Aufnehmen eines der Stäbe (100, 101) und
einen Kopfaufnahmeteil (17) zum Aufnehmen eines Kopfs (3) des Knochenverankerungselements, wobei der Kopfaufnahmeteil ein offenes Ende (19, 17b) hat und flexibel ist, um die Einführung und Klemmung des Kopfs zu erlauben; und
einen Verriegelungsring (8), der den Kopfaufnahmeteil (17) umgreift, wobei der Kopf durch Drücken auf den Verriegelungsring mit dem Stab geklemmt ist, um den Verriegelungsring in Richtung des offenen Endes (17b) zu bewegen;
wobei der Verriegelungsring (8) einen Oberflächenteil (86a, 86a') hat, der ausgebildet ist, um einen der Stäbe (100, 101) in einer Umlaufrichtung an mindestens zwei verschiedenen Kontaktbereichen (P 1, P2) zu berühren.

2. Die Stabilisierungsvorrichtung aus Anspruch 1, wobei der Oberflächenteil so geformt ist, dass die Kontaktbereiche (P1, P2) sich entlang der Stabachse.(R) erstrecken.

3. Die Stabilisierungsvorrichtung aus Anspruch 1 oder 2, wobei der Oberflächenteil (86a) kurvenförmig ist mit einem Kurvenradius, der kleiner ist als der Kurvenradius von jedem der Stäbe (100, 101).

4. Die Stabilisierungsvorrichtung aus einem der Ansprüche 1 bis 3, wobei der Oberflächenteil (86a) durch eine zylindrische Ausnehmung gebildet ist mit einem Durchmesser, der kleiner ist als der Durchmesser eines der Stäbe (100, 101).

5. Die Stabilisierungsvorrichtung aus einem der Ansprüche 1 bis 3, wobei der Oberflächenteil (86a') im Wesentlichen V-förmig ist.

6. Die Stabilisierungsvorrichtung aus Anspruch 5, wobei die Seitenwände (860a', 864a') der Basis, die die V-Form bildet, kurvenförmig sind.

7. Die Stabilisierungsvorrichtung aus Anspruch 5 oder 6, wobei der Oberflächenteil weiter gerade Seitenwände (863a') umfasst.

8. Die Stabilisierungsvorrichtung aus einem der Ansprüche 1 bis 7, wobei der Oberflächenteil derart geformt ist, um eine Mehrzahl von Stäben mit verschiedenen Durchmessern auf stufenlose Weise zu klemmen.

9. Die Stabilisierungsvorrichtung aus einem der Ansprüche 1 bis 8, wobei der Stab einen im Wesentlichen kreisförmigen Querschnitt hat.

10. Die Stabilisierungsvorrichtung aus einem der Ansprüche 1 bis 9, wobei der Verriegelungsring zwei Vorsprünge (86) hat, die 180° versetzt voneinander sind und wobei jede der Vorsprünge den Oberflächenteil aufweist, der ausgebildet ist, um jeden der Stäbe in einer Umlaufrichtung an mindestens zwei verschiedenen Kontaktbereichen zu berühren.

11. Die Stabilisierungsvorrichtung aus Anspruch 10, wobei die Vorsprünge (86) sich in den Kanal (12) erstrecken.

## Revendications

1. Dispositif de stabilisation permettant de stabiliser des vertèbres ou d'autres os comportant
au moins deux tiges (100, 101) ayant un diamètre différent ;
un élément d'ancrage (1) ayant un arbre (2) pour s'ancrer dans l'os et une tête (3) ;
une partie de réception (5) pour relier une tige parmi l'au moins deux tiges à l'élément d'ancrage ;
la partie de réception comprenant
une extrémité supérieure (9a) et une extrémité inférieure (17b) ;
une partie (9) de réception de tige avec un canal (12) pour recevoir l'une des tiges (100, 101) ; et
une partie (17) de réception de tête pour recevoir une tête (3) de l'élément d'ancrage osseux, la partie de réception de tête présentant une extrémité ouverte (19, 17b) et étant flexible de manière à permettre l'introduction et le serrage de la tête ; et
une bague de verrouillage (8) entourant la partie (17) de réception de tête, où la tête est serrée en appuyant sur la bague de verrouillage avec la tige pour déplacer la bague de verrouillage vers l'extrémité ouverte (17b) ;
où la bague de verrouillage (8) présente une partie de surface (86a, 86a') configurée pour entrer en contact avec l'une des tiges (100, 101) dans une direction circonférentielle au niveau d'au moins deux zones de contact distinctes (P1, P2).

2. Dispositif de stabilisation de la revendication 1, dans lequel la partie de surface est formée de sorte que les zones de contact (P1, P2) s'étendent le long de l'axe de tige (R).

3. Dispositif de stabilisation de la revendication 1 ou 2, dans lequel la partie de surface (86a) est courbée avec un rayon de courbure qui est inférieur à un rayon de courbure de chacune des tiges (100, 101).

4. Dispositif de stabilisation de l'une des revendications 1 à 3, dans lequel la partie de surface (86a) est formée par un évidement cylindrique avec un diamètre inférieur au diamètre de l'une des tiges (100, 101).

5. Dispositif de stabilisation de l'une des revendications 1 à 3, dans lequel la partie de surface (86a') est essentiellement en forme de V.

6. Dispositif de stabilisation de la revendication 5, dans lequel les parois latérales (860a', 864a') de la base qui forment la forme en V sont courbées.

7. Dispositif de stabilisation de la revendication 5 ou 6, dans lequel la partie de surface comporte en outre des parois latérales droites (863a').

8. Dispositif de stabilisation de l'une des revendications 1 à 7, dans lequel la partie de surface est formée de manière à serrer une pluralité de tiges de diamètres différents en continu.

9. Dispositif de stabilisation de l'une des revendications 1 à 8, dans lequel la tige présente une section transversale essentiellement circulaire.

10. Dispositif de stabilisation de l'une des revendications 1 à 9, dans lequel la bague de verrouillage comprend deux saillies (86) qui sont décalées de 180° l'une de l'autre et où chacune des saillies comprend la partie de surface configurée pour entrer en contact avec chacune des tiges dans une direction circonférentielle au niveau d'au moins deux zones de contact distinctes.

11. Dispositif de stabilisation de la revendication 10, dans lequel les saillies (86) s'étendent dans le canal (12).
